# EUROPEAN PATENT SPECIFICATION

(11) **EP 2 037 889 B1**
(45) Date of publication and mention of the grant of the patent: **20.10.2010**
(21) Application number: 07764871.5
(22) Date of filing: 26.06.2007
(51) Int. Cl.: A61K 9/19, A61K 31/428, A61K 31/425

(54) **NANOSTRUCTURED LIPID CARRIERS CONTAINING RILUZOLE AND PHARMACEUTICAL FORMULATIONS CONTAINING SAID PARTICLES**
NANOSTRUKTURIERTE LIPIDTRÄGER MIT RILUZOL UND DIESE TEILCHEN ENTHALTENDE PHARMAZEUTISCHE FORMULIERUNGEN
VECTEURS LIPIDIQUES NANOSTRUCTURÉS CONTENANT DU RILUZOLE ET FORMULES PHARMACEUTIQUES CONTENANT LESDITES PARTICULES

(30) Priority: 30.06.2006 IT MI20061274
(43) Date of publication of application: 25.03.2009
(73) Proprietor: CONSIGLIO NAZIONALE DELLE RICERCHE, 00185 Roma (IT); Universita' Degli Studi Di Catania, 95124 Catania (IT); Universita' degli Studi di Palermo, 90133 Palermo (IT)
(72) Inventor: BONDI', Maria, Luisa, I-90010 Altavilla Milicia (IT); GIAMMONA, Gaetano, I-90146 Palermo (IT); CRAPARO, Emanuela, Fabiola, 92019 Sciacca (IT); DRAGO, Filippo, I-95125 Catania (IT)
(74) Representative: Minoja, Fabrizio
(86) International application number: PCT/EP2007/005654
(87) International publication number: WO 2008/000448

(56) References cited:
- WO-A-03/083067
- WO-A-2004/096216
- US-A1- 2006 122 113

## Description

The present invention relates to nanoparticulate lipid vectors containing riluzole, and the preparation and characterisation thereof. The systems obtained have a different biodistribution from the free drug *in vivo,* and can be used to prepare pharmaceutical formulations.

### STATE OF THE ART

In view of their biodegradability and their ability to trap a variety of biologically active compounds, solid lipid nanoparticles are suitable for use as systems for modified drug release. For example, lipid nanoparticles have certain advantages over other colloidal vectors, such as the use of physiological lipids, the absence of organic solvents in the preparation process, a broad spectrum of applications, and the possibility of manufacture on a large scale at low cost. These systems are also able to modify the pharmacokinetics and biodistribution *in vivo* of the drugs trapped in them. Lipid nanoparticles can be used by various administration routes. Unfortunately, as these systems consist of pure lipids, some of them tend to crystallise, with the result that their drug loading is rather limited, and they tend to expel the drug with time.

It is therefore necessary to develop materials based on lipids which are already used in the pharmaceutical field as excipients, but which do not crystallise when mixed together. Systems are thus obtained wherein the lipid matrix is amorphous, which means that larger amounts of the drug can be trapped, and it is more stable over time.

### DESCRIPTION OF THE INVENTION

It has now been found that if lipids are used, in particular mixtures of glycerides and behenic acid in which riluzole, the drug currently used in the treatment of Amyotrophic Lateral Sclerosis (ALS) and Multiple Sclerosis (MS), is trapped, spherical nanoparticles with a diameter of under 100 nm can be obtained, which are able to deliver a larger quantity of riluzole to the central nervous system than the free form of the drug. Moreover, the drug carried accumulates in the non-target organs to a much lower extent than the free drug. All this demonstrates that the use of the carrier not only increases the targeting of the drug, but also reduces the side effects caused by indiscriminate distribution.

### DESCRIPTION OF THE INVENTION

A mixture of mono-, di- and triglycerides with behenic acid of plant origin is currently used in pharmaceutical technology as a lubricant for tablets, and is available on the market under the Compritol® brand. Said mixture is melted at approx. 85°C, and between 5 mg and 150 mg of riluzole is added to the molten mass. A hot microemulsion is therefore obtained with the use of surfactants such as phosphatidylcholine and co-surfactants such as taurocholic acid sodium salt. The hot microemulsion is then dispersed in cold water under agitation. The nanoparticles obtained are purified by ultracentrifugation to eliminate the surfactants used to prepare the microemulsion, and then freeze-dried.

The nanoparticulate systems thus obtained have been characterised in terms of size and polydispersity index using the Photon Correlation Spectroscopy (PCS) technique. The nanoparticles were dispersed in bidistilled water, saline solution (NaCl 0.9%), and phosphate buffer at pH 7.4. The dimensions were under 100 nm, with a polydispersity index of 0.25. They were also characterised by the Transmission Electron Microscopy (TEM) technique, which demonstrated the spherical form of the particles and confirmed the dimensions obtained with the PCS technique.

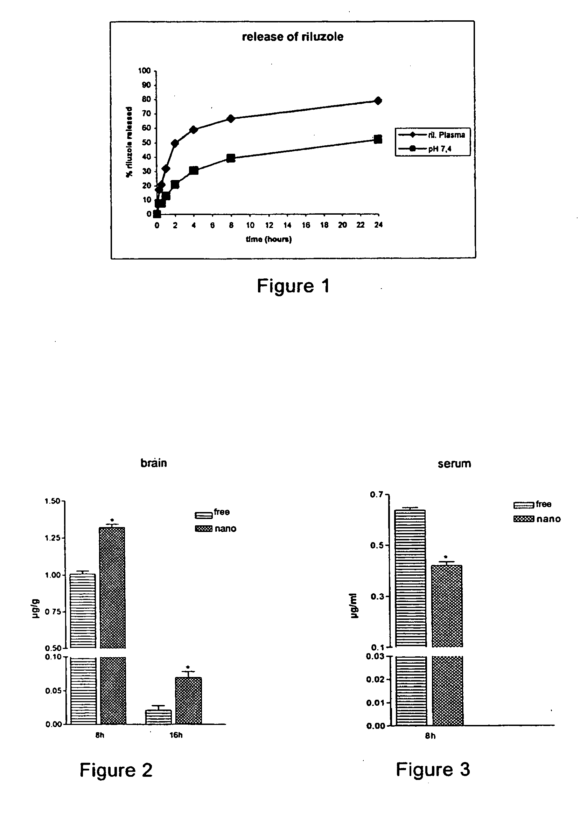

The system according to the invention was subjected to *in vitro* release studies at 37°C using media simulating some biological fluids (pH range analysed between 1 and 7.4) with incubation times of between 0.25 and 72 hours. The results demonstrated that the system according to the invention releases the drug slowly, up to a maximum of 75% within 72 hours. Release studies conducted in human plasma demonstrated that 79% of the drug is released within 24 hours (Figure 1).

The system according to the invention was subjected to *in vivo* studies on rats to evaluate the differences in biodistribution between the drug carried by the nanoparticles and the free drug. The trial was also designed to establish whether the neuroprotective action of the drug delivered by the system was more effective than that of the free drug. In particular, tests were performed to establish whether the rats treated with the carried riluzole manifested clinical signs of allergic encephalomyelitis (EAE), the experimental model of Multiple Sclerosis (MS), more slowly than those treated with free riluzole. The results demonstrated that the nanoparticulate systems according to the invention cross the blood-brain barrier (BBB) more easily, thus enabling the drug to reach higher concentrations in the central nervous system. These results have been verified on rats in both acute and chronic tests.

### EXAMPLE

The experiments were carried out on adult male Sprague Dawley rats weighing 250-300 g. After being housed in the animal unit for one week, the rats were immunised using the experimental allergic encephalomyelitis (EAE) model. For this purpose, Mycobacterium tuberculosis strain H37RA was inoculated subcutaneously, at the base of the tail. Two formulations were used for the treatment: the free drug and the drug trapped in nanoparticulate lipid carriers. A drug loading of 14.5% was calculated by HPLC for the nanoparticulate lipid batch containing riluzole. The two formulations were administered by the intraperitoneal route, dissolved in the vehicle (20% Tween 80) at the dose of 8 mg/kg of body weight.

As riluzole has a neuroprotective action, it was decided to administer it as a pre-treatment, ie. before the appearance of clinical signs of EAE., which are usually observed from the 14th day after the disease is induced. The animals were divided into two groups, each of which received, intraperitoneally, one of the two formulations described above. The treatment began on the 7th day after immunisation. At this preventive stage, the blood-brain barrier is believed to be still intact, because the inflammatory process is absent. Four animals were used for each data-point.

8, 16 and 30 hours after administration, the rats were sacrificed, and blood, brain, liver, spleen, heart, lung and kidney samples were taken.

The riluzole levels were determined by HPLC. The drug was extracted from the serum by adding 1 ml of CH₃CN to 50 µl of serum. The mixture was stirred and then centrifuged at 10,000 rpm for 15 min. The extraction was repeated with 1 ml of CH₃CN. The organic extract was filtered through 0.45 µm nylon and injected into the HPLC system. After deproteinisation, the riluzole was extracted from the brain and other organs. Each organ was weighed, and homogenised with 2 ml of Tris 1 M buffer, pH 8.5. The mixture was subjected to liquid-liquid extraction with CH₃CN and maintained at ambient temperature for 15 min. The precipitated proteins were removed by centrifugation at 10.000 rpm at 4°C. The organic extract was assayed by HPLC after filtration through an 0.45 µm nylon filter.

The results demonstrated that the rats treated with riluzole trapped in the nanoparticulate system according to the invention developed clinical signs of allergic encephalomyelitis later than those treated with free riluzole. The results indicated that the efficacy of the riluzole trapped in the system according to the invention was significantly greater than the efficacy of free riluzole. The riluzole in the lipid nanoparticles crossed the blood-brain barrier more easily than the free riluzole, and reached higher concentrations in the central nervous system, leading to a significant increase in its efficacy (Figures 2-3).

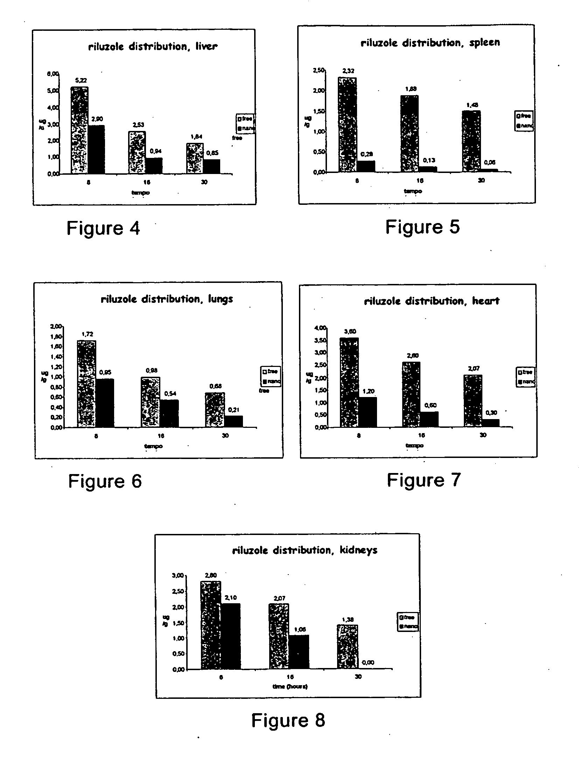

There was also a smaller accumulation of trapped riluzole than free riluzole in the liver, spleen, heart, kidneys and lungs, demonstrating that the drug delivered by the system is less toxic than the free drug (Figures 4-8).

## Claims

1. Nanoparticles consisting of riluzole trapped in lipids.

2. Nanoparticles as claimed in claim 1, wherein the lipids consist of mixtures of glycerides with behenic acid.

3. Nanoparticles as claimed in claim 1 or 2 of spherical shape, with a diameter of under 100 nm.

4. Pharmaceutical compositions containing the nanoparticles claimed in claims 1-3.

5. Use of the nanoparticles claimed in claims 1 to 3 for the preparation of medicaments for the treatment of Amyotrophic Lateral Sclerosis (ALS) and Multiple Sclerosis.

## Patentansprüche

1. Nanopartikel, die aus in Lipiden eingeschlossenem Riluzol bestehen.

2. Nanopartikel, wie in Anspruch 1 beansprucht, wobei die Lipide aus Gemischen von Glyceriden mit Behensäure bestehen.

3. Nanopartikel, wie in Anspruch 1 oder 2 beansprucht, mit Kugelform und einem Durchmesser von unter 100 nm.

4. Pharmazeutische Zusammensetzungen, die die in Ansprüche 1-3 beanspruchten Nanopartikel enthalten.

5. Verwerdung der in den Ansprüche 1-3 beanspruchten Nanopartikel für die Herstellung von Medikamenten für die Behandlung von Amyotropher Lateralsklerose (ALS) und Multipler Sklerose.

## Revendications

1. Nanoparticules constituées de riluzole piégé dans des lipides.

2. Nanoparticules selon la revendication 1, dans lesquelles les lipides sont constitués de mélanges de glycérides avec de l'acide béhénique.

3. Nanoparticules selon la revendication 1 ou 2, de forme sphérique, avec un diamètre inférieur à 100 nm.

4. Compositions pharmaceutiques contenant les nanoparticles telles que revendiquées dans les revendications 1 à 3.

5. Utilisation des nanoparticules revendiquées dans les revendications 1 à 3 pour la préparation de médicaments pour le traitement de la sclérose latérale amyotrophique (SLA) et de la sclérose en plaques.
